(19) Europäisches Patentamt
European Patent Office
Office européen des brevets

(11) Numéro de publication: **0 098 204**
**A1**

(12) **DEMANDE DE BREVET EUROPEEN**

(21) Numéro de dépôt: **83401266.8**

(22) Date de dépôt: **17.06.83**

(51) Int. Cl.³: **A 61 K 31/425**
A 61 K 31/15, A 61 K 31/155
A 61 K 31/435, A 61 K 31/495
A 61 K 31/535, C 07 D 277/50
C 07 D 417/12, C 07 D 417/14

(30) Priorité: **18.06.82 FR 8210687**

(43) Date de publication de la demande:
**11.01.84 Bulletin 84/2**

(84) Etats contractants désignés:
**AT BE CH DE FR GB IT LI LU NL SE**

(71) Demandeur: **LABORATOIRES CHAUVIN-BLACHE**
**Société dite:**
**104 Rue de la Galéra**
**F-34000 Montpellier(FR)**

(72) Inventeur: **Coquelet, Claude**
**113 Rue des Lilas**
**F-34980 Saint Gely du Fesc(FR)**

(72) Inventeur: **Bertez, Chantal Résidence"Le Languedoc"Bât. A4**
**Rue des Etats du Languedoc**
**F-34000 Montpellier(FR)**

(72) Inventeur: **Bonne, Claude**
**12 bis Avenue Aristide Briand**
**F-94360 Bry-sur-Marne(FR)**

(72) Inventeur: **Syncholle, Daniel**
**343 Avenue de la Trémoulette**
**F-34980 Saint-Clement(FR)**

(74) Mandataire: **Polus, Camille et al,**
**c/o Cabinet Lavoix 2, Place d'Estienne d'Orves**
**F-75441 Paris Cedex 09(FR)**

(54) **Compositions thérapeutiques à base d'hydrazones N-substituées et nouvelles hydrazones N-substituées.**

(57) L'invention a pour objet des compositions thérapeutiques contenant à titre de principe actif des hydrazones N-substituées de formule

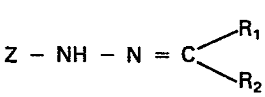 (I)

dans laquelle Z représente un groupe thiazolyle-2 ou phényle

$R_1$ représente un atome d'hydrogène, un groupe alkyle en $C_1$-$C_6$, un groupe cycloalkyle en $C_5$-$C_7$, un groupe hydroxy, un groupe alkoxy en $C_1$-$C_6$, un groupe carboxy, un groupe carboxy (alkyle en $C_1$-$C_6$), un groupe imidazolyl-1 (alkyle en $C_1$-$C_{10}$), un groupe de formule $-NHR_3$, $R_3$ étant un atome d'hydrogène, un groupe alkyle en $C_1$-$C_6$, un groupe cycloalkyle en $C_5$-$C_7$, un groupe amino (alkyle en $C_1$-$C_6$) ou un groupe hétérocyclique azoté non aromatique pouvant contenir un autre hétéroatome choisis parmi l'azote et l'oxygène ayant de 5 à 7 chaînons, ou un groupe de formule

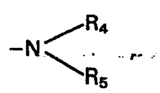

$R_4$ et $R_5$ formant avec l'atome d'azote sur lequel ils sont fixés un groupe hétérocyclique non aromatique ayant de 5 à 7 chaînons pouvant contenir un autre hétéroatome choisi parmi l'azote et l'oxygène et pouvant être substitué par un groupe alkyle en $C_1$-$C_6$,

$R_2$ représente un groupe carboxy (alkyle en $C_1$-$C_6$), un groupe (alkoxy en $C_1$-$C_6$) carbonyl (alkyle en $C_1$-$C_6$), un groupe aryle ayant de 6 à 16 atomes de carbone ou un groupe hétéroaromatique comprenant un ou deux hétéroatomes choisis parmi l'azote, l'oxygène et le soufre et de 3 à 15 atomes de carbone, les groupes aryle et hétéroaromatique pouvant être substitués par un ou plusieur groupes hydroxy, amino, carboxy ou carboxy (alkyle en $C_1$-$C_6$) ou bien

./...

EP 0 098 204 A1

$R_1$ et $R_2$ forment avec l'atome de carbone sur lequel ils sont fixés un radical choisi parmi les radicaux hydrocarbonés aromatiques bi- ou polycycliques ayant de 8 à 16 atomes de carbone, les radicaux hydrocarbonés mono- ou polycycliques non aromatiques ayant de 5 à 16 atomes de carbone et les radicaux hétéroaromatiques bi- ou polycycliques comprenant un ou deux hétéroatomes choisis parmi l'azote, l'oxygène et le soufre et de 6 à 15 atomes de carbone, ces radicaux pouvant être substitués par un groupe carboxy, ou l'un de ses sels d'addition avec un acide pharmaceutiquement acceptable.

Ces compositions ont une activité anti-inflammatoire, notamment dans le domaine oculaire.

# Compositions thérapeutiques à base d'hydrazones N-substituées et nouvelles hydrazones N-substituées.-

La présente invention concerne de nouvelles compositions thérapeutiques contenant à titre de principe actif des hydrazones N-substituées. Ces compositions peuvent être utilisées dans le traitement des phénomènes inflammatoires et notamment dans le traitement des phénomènes inflammatoires oculaires.

La réponse inflammatoire oculaire est la séquence des évènements entraînés par une lésion ou une irritation des tissus de l'oeil. Elle se traduit par l'augmentation de la pression intraoculaire, une infiltration leucocytaire dans les tissus et les liquides de l'oeil, une augmentation du taux de protéines dans l'humeur aqueuse et une uvéite.

Bien que la nature de la réponse inflammatoire soit extrêmement variée et ses médiateurs multiples (kinines, histamine, etc.), il y a toujours production de métabolites de l'acide arachidonique proinflammatoires au niveau du tissu lésé, à savoir prostaglandines ($PG_s$) et hydroxy-acides eicosatétraénoïques ($HETE_s$). Cette production est augmentée par l'infiltration des leucocytes polymorphonucléaires ($PMN_s$) vers le lieu de l'inflammation, qui produisent à leur tour durant la phagocytose des $PG_s$ et des $HETE_s$. Les effets biologiques connus des $HETE_s$ sont, entre autres, le chimiocinétisme et le chimiotactisme vis-à-vis des $PMN_s$.

Les $PG_s$ et les $HETE_s$ sont des métabolites de l'acide arachidonique, acide gras en $C_{20}$. Ils sont synthétisés selon deux voies distinctes qui sont schématisées sur la Fig. 1 :

- la voie de la cyclooxygénase conduit successivement aux endoperoxydes $PGG_2$ et $PGH_2$, précurseurs des divers $PG_s$ de la série 2. Parmi celles-ci, $PGE_2$ et $PGI_2$ (prostacycline) sont particulièrement vasodilatatrices.

- la voie de la lipoxygénase conduit aux acides non cyclisés mono- et dihydroxyeicosatétraénoïques ($HETE_s$) qui sont pour la plupart des agents proinflammatoires, comme leurs précurseurs, les acides hydroxyperoxylés correspondants ($HPETE_s$). Ainsi, le 5,12-diHETE ou leucotriène $B_4$ ($LTB_4$) est l'agent chimiotactique le plus puissant connu pour les leucocytes humains. Des métabolites de cette voie sont impliqués en outre dans les réactions allergiques telles que l'asthme.

Contrairement aux $PG_s$ qui ont des précurseurs communs, $PGG_2$ et $PGH_2$, les produits de lipoxygénase ont des précurseurs différents :

- le 12-HPETE dans les plaquettes, synthétisé sous l'action d'une 12-lipoxygénase, conduit à la formation de 12-HETE.
- dans les leucocytes, 5-HPETE et 15-HPETE sont principalement invoqués, conduisant aux 5-HETE, 5,12-diHETE et 15-HETE.

La recherche d'agents thérapeutiques susceptibles de s'opposer à la réaction inflammatoire était orientée vers les voies suivantes :

- inhibition de la libération d'acide arachidonique par les glucocorticoïdes. L'inconvénient majeur des glucocorticoïdes est de provoquer une augmentation de la pression intraoculaire et de favoriser l'apparition d'un glaucome ou d'une cataracte.
- inhibition de la voie de la cyclooxygénase par des antiinflammatoires non stéroïdiens tels que (Aspirine, Indométhacine, Tandéril). Mais des études expérimentales récentes montrent que l'Indométhacine augmente l'infiltration leucocytaire dans l'humeur aqueuse : en inhibant la voie de la cyclooxygénase, elle favorise la voie de la lipoxygénase et donc la libération de produits chimiotactiques.

L'inhibition simultanée de la voie de la 5-lipoxygénase et de la cyclooxygénase est susceptible de bloquer la synthèse des différents médiateurs pro-inflammatoires comme le font les glucocorticoïdes mais sans en présenter les effets gênants.

La présente invention est précisément basée sur la découverte que certaines hydrazones inhibent à la fois la lipoxygénase leucocytaire et la formation des prostanoïdes plaquettaires. Elles s'opposent donc au développement de la phase vasculaire de l'inflammation (impliquant les $HETE_s$).

La présente invention a ainsi pour objet des compositions thérapeutiques qui contiennent à titre de principe actif un composé de formule

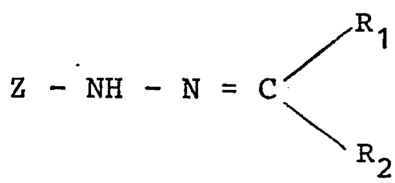

(I)

dans laquelle

Z représente un groupe thiazolyle-2 ou phényle

$R_1$ représente un atome d'hydrogène, un groupe alkyle en $C_1$-$C_6$, un groupe cycloalkyle en $C_5$-$C_7$, un groupe hydroxy, un groupe alkoxy en $C_1$-$C_6$, un groupe carboxy, un groupe carboxy (alkyle en $C_1$-$C_6$), un groupe imidazolyl-1 (alkyle en $C_1$-$C_{10}$), un groupe de formule $-NHR_3$, $R_3$ étant un atome d'hydrogène, un groupe alkyle en $C_1$-$C_6$, un groupe cycloalkyle en $C_5$-$C_7$, un groupe amino (alkyle en $C_1$-$C_6$) ou un groupe hétérocyclique azoté non aromatique pouvant contenir un autre hétéroatome choisi parmi l'azote et l'oxygène et ayant de 5 à 7 chaînons, ou un groupe de formule $-N\genfrac{}{}{0pt}{}{R_4}{R_5}$, $R_4$ et $R_5$ formant avec l'atome d'azote sur lequel ils sont fixés un groupe hétérocyclique non aromatique ayant de 5 à 7 chaînons, pouvant contenir un autre hétéroatome choisi parmi l'azote et l'oxygène et pouvant être substitué par un groupe alkyle en $C_1$-$C_6$ ,

$R_2$ représente un groupe carboxy (alkyle en $C_1$-$C_6$), un groupe (alkoxy en $C_1$-$C_6$) carbonyl (alkyle en $C_1$-$C_6$), un groupe aryle ayant de 6 à 16 atomes de carbone ou un groupe hétéroaromatique comprenant un ou deux hétéroatomes choisis parmi l'azote, l'oxygène et le soufre et de 3 à 15 atomes de carbone,
les groupes aryle et hétéroaromatique pouvant être substitués par un ou plusieur groupes hydroxy, amino, carboxy ou carboxy (alkyle en $C_1$-$C_6$), ou bien

$R_1$ et $R_2$ forment avec l'atome de carbone sur lequel ils sont fixés un radical choisi parmi les radicaux hydrocarbonés aromatiques bi- ou polycycliques ayant de 8 à 16 atomes de carbone, les radicaux hydrocarbonés mono- ou polycycliques non aromatiques ayant de 5 à 16 atomes de carbone et les radicaux hétéroaromatiques bi- ou polycycliques comprenant un ou deux hétéroatomes choisis parmi l'azote, l'oxygène et le soufre et de 6 à 15 atomes de carbone , ces radicaux pouvant être substitués par un groupe carboxy,
ou l'un de ses sels d'addition avec un acide pharmaceutiquement acceptable.

Dans la définition ci-dessus on désigne par "sels d'addition avec des acides pharmaceutiquement acceptables" les sels qui possèdent les propriétés biologiques des bases libres, sans avoir d'effet indésirable. Ces sels peuvent être notamment ceux formés avec des acides minéraux tels que l'acide chlorhydrique, l'acide bromhydrique, l'acide sulfurique, l'acide nitrique, l'acide phosphorique, des sels métalliques acides tels que l'orthophosphate disodique et le sulfate monopotassique, et des acides organiques tels que l'acide formique, l'acide acétique, l'acide propionique, l'acide glycolique, l'acide oxalique, l'acide fumarique, l'acide citrique, l'acide malique, l'acide méthane sulfonique, l'acide lactique, l'acide succinique, l'acide tartrique et l'acide pamoïque.

Le terme "halogène" désigne le chlore, le brome ou l'iode.

Par "radicaux hydrocarbonés aromatiques bi- ou polycycliques" on désigne des radicaux bi- ou polycycliques ayant au moins un cycle aromatique. Comme exemples de tels radicaux, on peut citer les radicaux tétrahydro-1,2,3,4-naphtylidène et indanylidène.

Par "radicaux hydrocarbonés mono- ou polycycliques non aromatiques" on désigne des radicaux hydrocarbonés cycliques ne comportant pas de cycle aromatique. Comme exemple de tels radicaux on peut citer le radical cyclohexylidène.

Par "radicaux hétéroaromatiques bi- ou polycycliques" on désigne des radicaux bi- ou polycycliques ayant au moins un cycle aromatique. Come exemples de tels radicaux, on peut citer les radicaux chromannylidène, chroménnylidène, phénylchromannylidène et indolinylidène.

Comme exemples de groupes hétérocycliques azotés non aromatiques entrant dans la définition de $R_3$ on peut citer les groupes pyrrolidinyle, imidazolinyle, pyrazolidinyle, pipéridyle, pipérazinyle ou morpholino.

Comme exemples de groupes hétérocycliques non aromatiques entrant dans la définition de $-NR_4R_5$ on peut citer les groupes pyrrolidinyle-1, pipéridino, pipérazinyle-1 ou morpholino.

Comme exemples de groupes hétéroaromatiques entrant dans la définition de $R_2$, on peut citer les groupes thiényle, furyle, pyrrolyle, pyridyle, imidazolyle, isoxazolyle.

Une classe préférée de composés de formule I est celle dans laquelle

Z présente un groupe thiazolyle-2

$R_1$ représente un groupe méthyle, et

$R_2$ représente un groupe (alkoxy en $C_1$-$C_6$) carbonyl (alkyle en $C_1$-$C_6$), un groupe thiényle ou un groupe phényle substitué par un ou plusieurs groupes hydroxy.

ou bien $R_1$ et $R_2$ foment avec l'atome de carbone sur lequel ils sont fixés un groupe chrommanylidène, indanylidène ou tétrahydro-1,2,3,4 naphtylidène.

Une autre classe préférée de composés de formule (I) est celle dans laquelle

Z représente un groupe phényle

$R_1$ représente un groupe de formule $-NHR_3$ ou $-NR_4R_5$, notamment un groupe amino ou un groupe méthylpipérazinyle, et

$R_2$ représente un groupe aryle ou hétéroaromatique, notamment un groupe phényle.

Un certain nombre de composés de formule (I) sont des composés connus (voir Chemische Berichte, vol. 85, n° 12, 1123-1127; Chemische Berichte, vol. 89, n° 5, 1095-1099; Talanta, vol. 27, 55-58; Bull. Soc. Chim. 1973, 9-10, 2843).

La présente invention a également pour objet des composés nouveaux de formule (I), à savoir les composés de formule

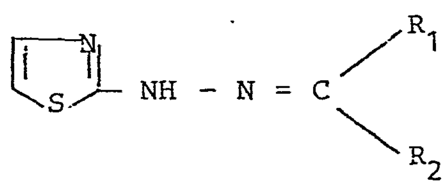

(I)

dans laquelle

$R_1$ représente un atome d'hydrogène, un groupe alkyle en $C_1$-$C_6$, un groupe cycloalkyle en $C_5$-$C_7$, un groupe hydroxy, un groupe alkoxy en $C_1$-$C_6$, un atome d'halogène, un groupe amino ou un groupe (alkyl en $C_1$-$C_6$) amino,

$R_2$ représente un groupe carboxy (alkyle en $C_1$ à $C_6$), un groupe (alkoxy en $C_1$-$C_6$) carbonyl (alkyle en $C_1$-$C_6$), un groupe thiényle, un groupe furyle, un groupe imidazolyl-1 (alkyle en $C_1$-$C_{10}$), un groupe phényle substitué par un ou plusieurs groupes hydroxy, ou bien

$R_1$ et $R_2$ forment avec l'atome de carbone sur

lequel ils sont fixés un radical choisi parmi les radicaux hydrocarbonés aromatiques bi- ou polycycliques ayant de 8 à 16 atomes de carbone, les radicaux hydrocarbonés mono- ou polycycliques non aromatiques ayant de 5 à 16 atomes de carbone et les radicaux hétéroaromatiques bi- ou polycycliques comprenant un ou deux hétéroatomes choisis parmi l'azote, l'oxygène et le soufre et de 6 à 15 atomes de carbone,
ainsi que leurs sels d'addition avec des acides pharmaceutiquement acceptables.

Les composés nouveaux et les composés connus peuvent être obtenus par des procédés classiques.

Ainsi, les composés de formule (I) dans laquelle $R_1$ est un atome d'hydrogène, un groupe alkyle ou cycloalkyle peuvent être préparés par réaction d'une hydrazine de formule

$$Z - NH - NH_2 \qquad \text{(II)}$$

sur une cétone de formule

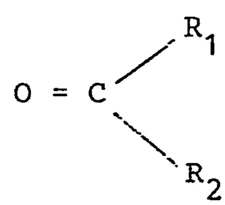

(III)

$R_1$ et $R_2$ ayant la définition donnée ci-dessus.

La réaction peut être effectuée notamment en solution dans un alcool, en présence d'une quantité catalytique d'un acide tel que l'acide acétique ou l'acide chlorhydrique. La réaction est effectuée généralement à température élevée, avantageusement à la température de reflux du solvant, en utilisant des quantités équimolaires d'hydrazine II et de cétone III et pendant une durée de 15 mn à 3 heures.

Dans le cas des cétones III comportant un groupe carboxy, on peut effectuer la réaction par chauffage au reflux de quantités équimolaires d'hydrazine II et de cétone III dans une solution aqueuse d'acide acétique à 10 % pendant 1 à 3 heures.

On peut également faire réagir l'hydrazine II sur la cétone III en ajoutant à la cétone III en solution dans un alcool une quantité équimolaire de chlorhydrate de thiazolyl-2 hydrazine en solution aqueuse. La réaction a lieu à température ambiante et peut être obtenue par exemple par agitation pendant 15 mn.

Les hydrazones de formule I dans lesquelles $R_1$ représente un groupe hydroxy, peuvent être préparées par réaction d'hydrazine II en solution dans la pyridine avec une quantité équimolaire de chlorure d'acide de formule

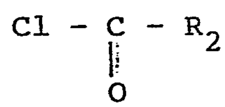

(IV)

La réaction peut être effectuée à 5°C sous agitation.

Les composés de formule (I) dans laquelle $R_1$ est un groupe alcoxy epuvent être obtenus à partir des composés de formule (I) dans laquelle $R_1$ est un groupe hydroxy, selon des méthodes classiques d'éthérification.

Les composés de formule I dans laquelle $R_1$ est un groupe $-NHR_3$ ou $-NR_4R_5$ peuvent être obtenus à partir d'un chlorure d'acide hydrazonique de formule

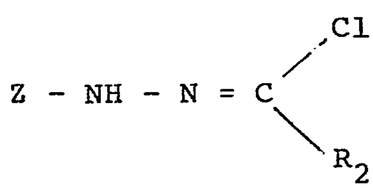

(V)

par addition d'une amine $-NH_2R_3$ ou $-NHR_4R_5$.

Les composés de formule (I) dans lesquels Z est un groupe thiazolyle-2 peuvent également être préparés par réaction d'une cétone III avec le thiosemicarbazide et réaction de la thiosemicarbazone obtenue avec du dichloro-1,2 éthoxy-2 éthane, du chloroacétaldéhyde, du chloroacétaldéhyde diéthylacétal ou de l'acétate de dichloro-1,2 éthyle, selon le schéma suivant:

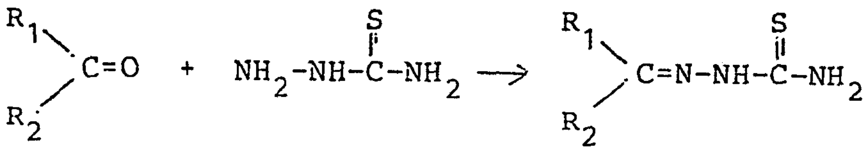

(III) (VI)

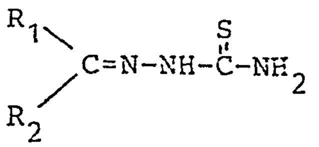

(VI)

+

$ClCH_2-CHCl-O-CH_2-CH_3$

$ClCH_2-CHO$

$ClCH_2-CH = (O\ CH_2\ CH_3)_2$

$ClCH_2-CHCl-O-CO-CH_3$

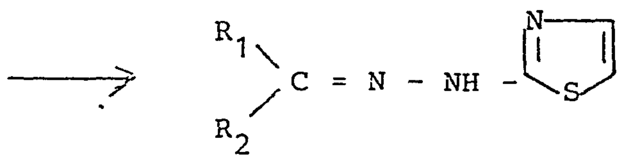

(Ia)

La réaction de la thiosémicarbazone (V avec par exemple le chloro acétaldéhyde diéthylacétal peut être effectuée en portant au reflux pendant une heure une solution alcoolique de quantités équimolaires des réactifs.

Les sels d'addition avec des acides pharmaceutiquement acceptables peuvent être préparés de manière classique par réaction des bases libres avec un acide ou un sel, notamment en solution dans un alcool.

On donnera ci-après dans les tableaux Ia et Ib des exemples de composés utilisés dans la présente invention. Ces composés ont été préparés selon les modes opératoires suivants :

- Mode opératoire A

On porte à ébullition pendant 15 minutes sous courant d'azote des quantités équimolaires de cétone III et d'hydrazine II dans une solution de méthanol contenant 5 % d'acide acétique.

On ajoute deux volumes d'eau, on triture, essore le précipité formé et recristallise le produit brut.

- Mode opératoire B

On ajoute à une solution refroidie à 5°C d'hydrazine II dans la pyridine une quantité équimolaire

de chlorure d'acide IV, on agite 15 minutes à 5°C et abandonne 48 heures à température ambiante. On concentre sous vide à moitié, on jette dans l'eau et essore le précipité. Après lavage par l'eau, le produit est recristallisé dans le méthanol.

- Mode opératoire C

On sature à froid une solution méthanolique d'hydrazone I par un courant d'HCl gazeux. On ajoute 5 volumes d'éther, essore le précipité formé et recristallise.

- Mode opératoire D

On porte à reflux 5 minutes des quantités équimolaires d'hydrazone I et d'acide dans du méthanol, on refroidit, ajoute un volume d'éther, essore le sel brut obtenu et le recristallise.

- Mode opératoire E

Dans une solution de 0,2 mole de chlorure d'acide hydrazonique de formule V dans du tétrahydrofuranne, on fait barboter à 0°C pendant 3 heures de l'ammoniac. Après avoir filtré le chlorure d'ammonium formé, le solvant est évaporé sous vide à température ambiante. Une solution éthérée de la base brute est saturée par un courant d'acide chlorhydrique gazeux. Le chlorhydrate est essoré, puis recristallisé.

- Mode opératoire F

A une solution benzénique de 0,2 mole de chlorure d'acide hydrazonique de formule V, on ajoute en 30 minutes à 5°C une solution benzénique d'une mole d'amine. L'agitation est continuée pendant 3 heures à 20°C, puis le chlorhydrate d'amine est éliminé par filtration. Le mode opératoire est ensuite identique à celui décrit ci-dessus.

- Mode opératoire G

Identique au précédent, mais l'addition est faite en 15 minutes à température ambiante, puis l'agitation est maintenue 1 heure à température ambiante et enfin 1 heure à reflux.

Le tableau Ia donne des exemples de composés de formule

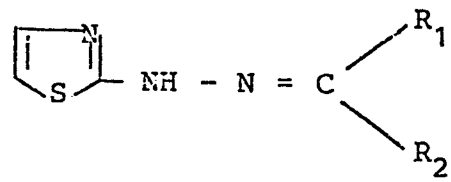

(Ia)

et le tableau Ib donne des exemples de composés de formule

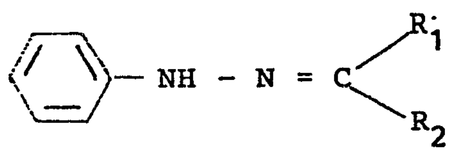

(Ib)

TABLEAU Ia

COMPOSES DE FORMULE Ia

| Exemple | 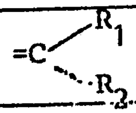 , sel | F (°C) solvant recrist. | Aspect des cristaux | Rendement % en poids | Mode opératoire |
|---|---|---|---|---|---|
| 1 | 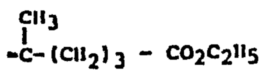 | 90 - 2<br>MeOH * | Jaune or | 56 | A |
| 1a | 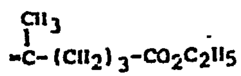 , HCl | 89 - 91<br>EtOH * | Incolore | 51 | C |
| 2 | 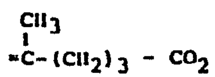 | 183 - 5<br>MeOH | Marron clair | 54 | A |
| 3 | 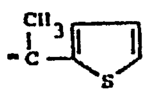 | 160 - 2<br>MeOH | Beige clair | 60 | A |
| 3a | 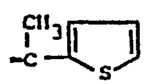 , HCl | 194 - 6<br>MeOH | Jaune | 56 | C |
| 3b | 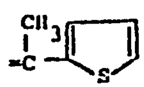 , 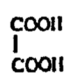 | 174 - 6<br>MeOH | Beige clair | 57 | D |
| 3c | 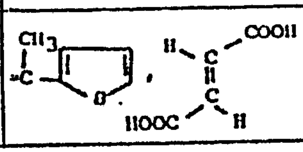 | 194 - 6<br>MeOH | Beige clair | 53 | D |

* MeOH = méthanol EtOH = éthanol 95°

| Exemple | R | F (°C) solvant recrist. | Aspect des cristaux | Rendement % en poids | Mode opératoire |
|---|---|---|---|---|---|
| 3d | 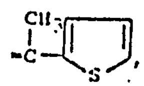 , 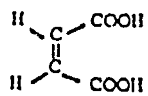 | 152 - 4 MeOH | Beige clair | 53 | D |
| 3e | 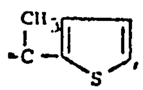 , 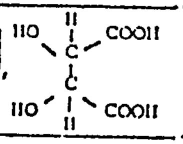 | 150 - 2 MeOH | Beige clair | 36 | D |
| 4 | 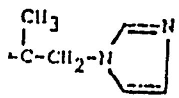 , HCl | 126 - 8 MeOH + $Et_2O$ * | Parme | 10 | C |
| 5 | 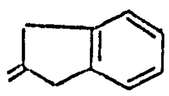 | 204 - 6 DMF * | Jaune paille | 56 | A |
| 5a | 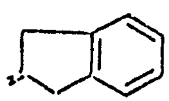 , HCl | 189 - 91 MeOH + $Et_2O$ | Incolore | 86 | C |
| 6 | 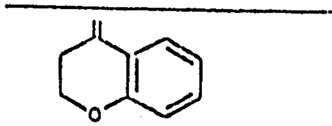 | 176 - 8 MeOH | Incolore | 30 | A |
| 6a | 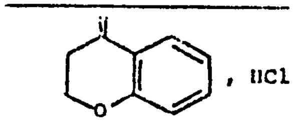 , HCl | 226° (dec.) MeOH | Incolore | 65 | C |

* $Et_2O$ = éther diéthylique DMF = diméthylformamide

| Exemple | R | F (°C) solvant recrist. | Aspect des cristaux | Rendement % en poids | Mode opératoire |
|---|---|---|---|---|---|
| 7 | 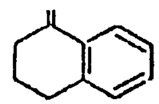 | 169 - 71 MeOH | Beige clair | 30 | A |
| 7a | 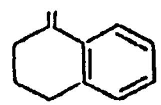 , HCl | 219 - 21 MeOH | Incolore | 72 | C |
| 8 | 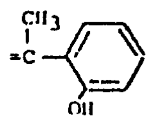 | 176 - 8 MeOH | Beige clair | 40 | A |
| 8a | 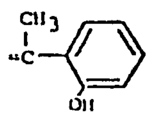 , HCl | 208 - 10 MeOH | Beige clair | 41 | C |
| 9 | 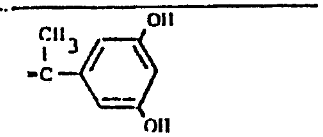 | 177 - 9 MeOH | Incolore | 30 | A |
| 9a | 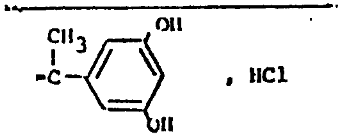 , HCl | 233 - 5 MeOH | Beige clair | 50 | C |
| 10 | 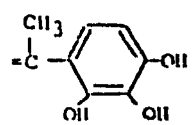 | 125 - 7 MeOH | Beige clair | 20 | A |

| Exemple | R | F (°C) solvant recrist. | Aspect des cristaux | Rendement % en poids | Mode opératoire |
|---|---|---|---|---|---|
| 10a | 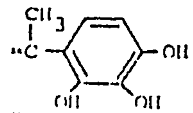 , HCl | 240 (dec.) | Beige clair | 50 | C |
| 11 | 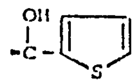 | 213 - 5 MeOH | Jaune brique | 20 | B |
| 11a | 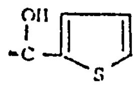 , HCl | 195 - 7 MeOH + $Et_2O$ | Beige clair | 53 | C |
| 12 | 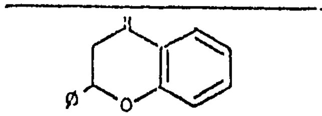 | 93 - 5 MeOH | Incolore | 30 | A |
| 12a | 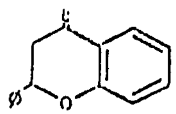 ,HCl | 119 - 21 MeOH + $Et_2O$ | Incolore | 50 | C |
| 13 | 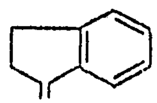 | 177 - 9 MeOH | Incolore | 57 | A |
| 13a | 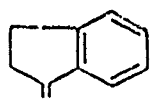 ,HCl | 239 - 41 MeOH | Jaune paille | 71 | C |

TABLEAU Ia (Suite)

COMPOSES DE FORMULE Ia

| Exemple | 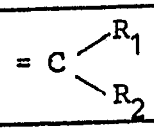 , sel | F (°C) Solvant recrist. | Aspect des cristaux | Rendement % en poids | Mode opératoire |
|---|---|---|---|---|---|
| 14 | 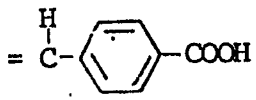 | > 250 MeOH | Jaune paille | 40 | A |
| 15 | 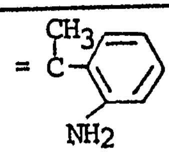 , HCl | 241 - 3 MeOH | Beige clair | 70 | A + C |
| 16 | 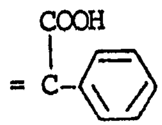 | 204 - 6 MeOH | Jaune or | 54 | A |
| 17 | 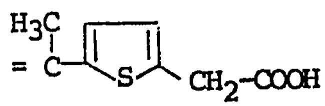 | 232 - 4 MeOH | Orange | 53 | A |
| 18 | 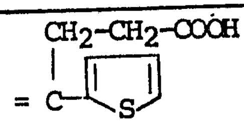 | 184 - 6 MeOH | Jaune paille | 45 | A |
| 19 | 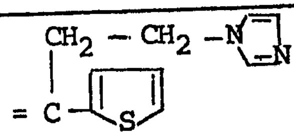 | 216 - 8 MeOH | Jaune paille | 15 | A |
| 20 | 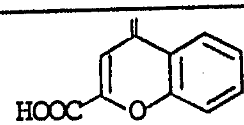 | 186 - 8 MeOH | Incolore | 57 | A |

TABLEAU Ib

COMPOSES DE FORMULE Ib

| Exemple | 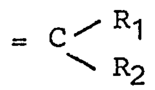 , sel | F (°C) Solvant recrist. | Aspect des cristaux | Rendement % en poids | Mode opératoire |
|---|---|---|---|---|---|
| 21 | 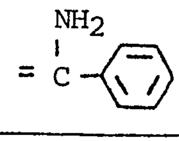 , HCl | 190 - 2 EtOH + $Et_2O$ | Incolore | 60 | E |
| 22 | 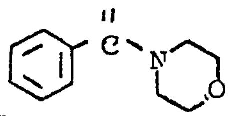 , HCl | 227 - 9 EtOH | Jaune paille | 40 | F |
| 23 | 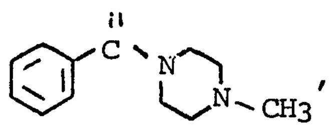 , HCl | 222 - 4 EtOH = $Et_2O$ | Incolore | 54 | F |
| 24 | 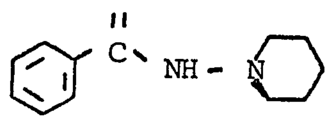 , HCl | 212 - 4 EtOH | Beige clair | 60 | F |
| 25 | 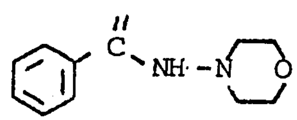 , HCl | 114 - 6 EtOH + $Et_2O$ | Incolore | 26 | G |

On donnera ci-après des résultats des études pharmacologiques et toxicologiques mettant en évidence les propriétés des composés de formule I.

1. PHARMACOLOGIE BIOCHIMIQUE "IN VITRO"

1.1. Inhibition de la 5-lipoxygénase des leucocytes péritonéaux de lapin ($PMN_s$)

Méthodes

. Préparation d'une suspension de $PMN_s$ de lapin.

Les leucocytes péritonéaux de lapin sont obtenus et préparés selon la technique décrite par P. Borgeat et B. Samuelson (J. Biol. Chem. 254,n°8, p.2643, 1979).

. Métabolisme du $^{14}C$-Arachidonate.

Les méthodes d'incubation et d'extraction sont celles décrites par J.Y. Vanderhoek et coll. (J.Biol. Chem. 255, 21, 10064, 1980).

Résultats

La Fig. 2a montre un profil chromatographique caractéristique du métabolisme du $^{14}C$-AA dans les $PMN_s$ péritonéaux de lapin comparé à celui obtenu en présence du composé de l'exemple 3b (50 µM) (Fig. 2b). Ce dernier tracé met en évidence l'inhibition du métabolisme de la voie 5-lipoxygénase.

Le pourcentage d'inhibition de la voie 5-lipoxygénase est mesuré en fonction de la concentration de chaque composé. De ces courbes, on déduit la concentration de produit inhibant 50 % du métabolisme ($IC_{50}$).

De nombreux composés de formule I inhibent à 50 % la formation de 5-HETE et de $LTB_4$ à une concentration comprise entre 2 et 20 µM.

A titre d'exemple, un certain nombre de résultats sont donnés dans le tableau II.

1.2. Inhibition du métabolisme du $^{14}C$-Arachidonate dans les plaquettes de lapin

Méthodes

. Préparation d'un plasma riche en plaquettes (PRP).

On prélève en intracardiaque 12 ml de sang sur ACD (anticoagulant constitué par une solution aqueuse contenant 0,27 g d'aciee citrique, 0,5 g de citrate disodique et 0,02 g de glucose pour 20 ml d'eau), à raison de 1 volume d'ACD pour 6 volumes de sang. Le sang est centrifugé 10 minutes à 200 g et le surnageant (PRP) est collecté. Il est recentrifugé 15 minutes à 1500 g. Le culot plaquettaire est lavé dans le Tampon I (NaCl : 8 g/l, Tris:1,21 g/l, KCl: 0,2 g/l, Gélatine : 2,5 g/l; on chauffe à 90°C pour dissoudre la gélatine, puis on ajoute glucose : 1 g/l, acide N,N'-éthylèneglycol bis (β-aminoéthyl éther tétraacétique ou EGTA : 0,075 g/l). La suspension est centrifugée 10 minutes à 1500 g et le culot est repris dans le Tampon II (NaCl : 8 g/l, Tris : 1,21 g/l, KCl : 0,2 g/l, $CaCl_2$ : 0,145 g/l, $MgCl_2$ : 0,2 g/l, gélatine : 2,5 g/l; on porte à 90°C, ajoute glucose: 1 g/l, ajuste à pH 7,4 avec HCl 0,1 N).

. Métabolisme du $^{14}$C-Arachidonate.

La suspension plaquettaire (0,4 ml/tube) est préincubée 15 minutes à 37°C, soit avec 10 µl de DMSO (témoin), soit avec des concentrations variées de produits à traiter en solution dans 10 µl de DMSO.

L'incubation est faite en présence de $^{14}$C-Arachidonate (1 µg, 0,2 µCi) réparti dans 0,1 ml de Tampon II. Elle est bloquée au bout de 15 minutes par 50 µl d'acide citrique et 250 µl de NaCl saturé.

. Résultats

TABLEAU II

| Exemple | $IC_{50}$ (x $10^{-6}$ M) dans les $PMN_s$ | | | $IC_{50}$ (x $10^{-6}$ M) dans les plaquettes | | |
|---|---|---|---|---|---|---|
| | $LTB_4$ | 5 HETE | 15 HETE | $TxB_2$ | HHT | 12 HETE |
| 1 | < 2 | < 2 | > 50 | > 50 | > 50 | > 50 |
| 1a | 13 | < 2 | n.d. | 12 | 50 | n.i. |
| 3 | 7 | 18 | n.i. | 50 | 20 | 3 |
| 3a | 18 | 15 | 40 | 13 | 21 | 30 |
| 3b | 13 | 10 | 40 | 10 | > 50 | 10 |
| 3c | 2 | 10 | > 50 | 10 | > 50 | 14 |
| 3d | 2 | < 2 | n.i. | 10 | 50 | 22 |
| 3e | < 2 | < 2 | n.d. | n.d. | n.d. | n.d. |
| 4 | n.i. | n.i. | 2 | > 50 | > 50 | > 50 |
| 7 | 8 | 6 | 2 | 10 | 16 | 40 |
| 7a | 6 | 5 | < 2 | 5 | 15 | n.i. |

n.i. = non inhibé

n.d. = non déterminé

TABLEAU II (Suite)

| Exemples | $IC_{50}$ (x $10^{-6}$ M) dans les $PMN_s$ | | | $IC_{50}$ (x $10^{-6}$ M) dans les plaquettes | | |
|---|---|---|---|---|---|---|
| | $LTB_4$ | 5 HETE | 15 HETE | $TxB_2$ | HHT | 12 HETE |
| 15 | > 25 | > 25 | n.d. | > 25 | 25 | n.i. |
| 20 | n.i. | n.i. | n.d. | 15 | 20 | n.i. |
| 21 | 10 | 20 | n.d. | 10 | 10 | n.i. |
| 23 | 25 | 25 | n.d. | 25 | 25 | 25 |

Le tracé chromatographique caractéristique du métabolisme du $^{14}C$-Arachidonate dans les plaquettes de lapin est modifié en présence d'inhibiteurs de la cyclooxygénase.

Les Fig. 3a et 4a donnent les profils chromatographiques du métabolisme du $^{14}C$-AA dans les plaquettes de lapin, comparé à celui obtenu en présence d'Aspirine (Fig. 3b) et du composé de l'exemple 3b (Fig. 4b).

Le pourcentage d'inhibition des voies des prostanoïdes et de la 12-lipoxygénase est estimé par mesure de la disparition du 12-HETE et du thromboxane $B_2$ ($TxB_2$) en fonction de concentrations variées de chaque composé.

Ce test met en évidence que les composés de formule I présentant une bonne activité sur la voie 5-lipoxygénase sont en général les plus actifs sur $TxB_2$.

Des résultats spécifiques sont donnés à titre d'exemple dans le tableau II.

On constate également au vu de la Fig. 3b que l'Aspirine en revanche n'inhibe pas la voie de 12-lipoxygénase.

2. PHARMACOLOGIE FONCTIONNELLE

Activité anti-inflammatoire cutanée mesurée par inhibition de l'oedème de l'oreille chez le rat après administration topique

Cette activité est mesurée selon la méthode de G. Tonelli et coll. (Endocrinology, 1965, 77, 625-634).

L'effet des diverses concentrations de chaque composé est mesuré sur au moins sept rats.

On inhibe l'inflammation à 50 % aux concentrations suivantes :

| | | | |
|---|---|---|---|
| Dexaméthasone | : | 1 mg/ml | ( 2,5 µmoles/ml) |
| Indométhacine | : | non inhibé | |
| Aspirine | : | 70 mg/ml | ( 233 µmoles/ml) |
| NDGA * | : | 12,5 mg/ml | ( 41 µmoles/ml) |
| Composé de l'exemple 3b | : | 16 mg/ml | ( 57 µmoles/ml) |

* Acide nordihydroguaïarétique.

3. TOXICITE AIGUË

Les souris utilisées sont de souche Swiss (NMRI-Han). Le poids moyen des animaux mâles est de 30 g, celui des femelles 25 g.

L'étude de la toxicité aiguë a été réalisée par voie intrapéritonéale.

Les composés en solution dans le DMSO sont injectés sous la face ventrale du péritoine, à des doses croissantes.

Le lot témoin reçoit du DMSO dans les mêmes conditions (2,5 $ml.kg^{-1}$).

La $DL_{50}$ est déterminée après 14 jours d'observation.

Résultats

Le tableau III ci-après donne les valeurs

des doses létales 50 % accompagnées de l'intervalle de confiance à 5 %.

On note une légère différence entre les sexes.

TABLEAU III

$DL_{50}$ parentérale du composé de l'exemple 3b chez la souris (mg.kg-1)

| Souris mâles | Souris femelles |
|---|---|
| 105 ± 24 | 155 ± 32 |

Les compositions thérapeutiques selon l'invention peuvent être administrées à l'homme ou aux animaux par voie topique, orale ou parentérale (y compris par voie intraarticulaire).

Elles peuvent être sous la forme de préparations solides, semi-solides ou liquides. Comme exemples, on peut citer les comprimés, les gélules, les suppositoires, les solutions ou suspensions injectables, les pommades, les collyres huileux ou aqueux, les collutoires, les solutions nasales et otologiques, ainsi que les formes retard.

Dans ces compositions le principe actif est généralement mélangé avec un ou plusieurs excipients pharmaceutiquement acceptables habituels bien connus de l'homme de l'art.

Les compositions thérapeutiques administrables par voie topique peuvent contenir notamment de 0,5 à 5 % en poids de principe actif.

Les compositions thérapeutiques administrables par voie orale ou parentérale peuvent contenir notamment de 1 à 60 % en poids de principe actif.

La quantité de principe acitf administrée dépend évidemment du patient qui est traité, de la voie

BAD ORIGINAL

d'administration et de la sévérité de la maladie. Cependant pour l'administration par la voie orale ou parentérale, on peut administrer environ de 0,25 à 5 mg/kg/jour, soit chez un homme de 70 kg de 17,5 à 350 mg/kg/jour et, de préférence, de 25 à 250 mg/jour.

Les composés de formule I et leurs sels d'addition avec des acides pharmaceutiquement acceptables sont également utilisables en raison de leurs propriétés antioxydantes comme conservateurs et antioxydants en alimentation humaine et animale.

REVENDICATIONS

1. Compositions thérapeutiques qui contiennent à titre de principe actif un composé de formule

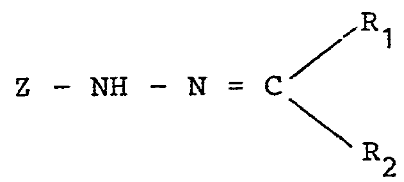

(I)

dans laquelle

Z représente un groupe thiazolyle-2 ou phényle

$R_1$ représente un atome d'hydrogène, un groupe alkyle en $C_1$-$C_6$, un groupe cycloalkyle en $C_5$-$C_7$, un groupe hydroxy, un groupe alkoxy en $C_1$-$C_6$, un groupe carboxy, un groupe carboxy (alkyle en $C_1$-$C_6$), un groupe imidazolyl-1 (alkyle en $C_1$-$C_{10}$), un groupe de formule $-NHR_3$, $R_3$ étant un atome d'hydrogène, un groupe alkyle en $C_1$-$C_6$, un groupe cycloalkyle en $C_5$-$C_7$, un groupe amino (alkyle en $C_1$-$C_6$) ou un groupe hétérocyclique azoté non aromatique pouvant contenir un autre hétéroatome choisis parmi l'azote et l'oxygène ayant de 5 à 7 chaînons, ou un groupe de formule 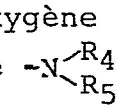 , $R_4$ et $R_5$ formant avec l'atome d'azote sur lequel ils sont fixés un groupe hétérocyclique non aromatique ayant de 5 à 7 chaînons pouvant contenir un autre hétéroatome choisi parmi l'azote et l'oxygène et pouvant être substitué par un groupe alkyle en $C_1$-$C_6$ ,

$R_2$ représente un groupe carboxy (alkyle en $C_1$-$C_6$), un groupe (alkoxy en $C_1$-$C_6$) carbonyl (alkyle en $C_1$-$C_6$), un groupe aryle ayant de 6 à 16 atomes de carbone ou un groupe hétéroaromatique comprenant un ou deux hétéroatomes choisis parmi l'azote, l'oxygène et le soufre et de 3 à 15 atomes de carbone,
les groupes aryle et hétéroaromatique pouvant être substitués par un ou plusieur groupes hydroxy, amino, carboxy ou carboxy (alkyle en $C_1$-$C_6$), ou bien

$R_1$ et $R_2$ forment avec l'atome de carbone sur lequel ils sont fixés un radical choisi parmi les radicaux hydrocarbonés aromatiques bi- ou polycycliques ayant de 8 à 16 atomes de carbone, les radicaux hydrocarbonés mono- ou polycycliques non aromatiques ayant de 5 à 16 atomes de carbone et les radicaux hétéroaromatiques bi- ou polycycliques comprenant un ou deux hétéroatomes choisis parmi l'azote, l'oxygène et le soufre et de 6 à 15 atomes de carbone , ces radicaux pouvant être substitués par un groupe carboxy,
ou l'un de ses sels d'addition avec un acide pharmaceutiquement acceptable.

2. Compositions selon la revendication 1, caractérisées en ce qu'elles contiennent à titre de principe actif un composé de formule I dans laquelle

Z représente un groupe thiazolyle-2

$R_1$ représente un groupe méthyle, et

$R_2$ représente un groupe (alkoxy en $C_1$-$C_6$) carbonyl (alkyle en $C_1$-$C_6$), un groupe thiényle ou un groupe phényle substitué par un ou plusieurs groupes hydroxy,

ou bien $R_1$ et $R_2$ forment avec l'atome de carbone sur lequel ils sont fixés un groupe chromannylidène, indanylidène ou tétrahydro-1,2,3,4-naphtylidène,
ou l'un de ses sels d'addition avec un acide pharmaceutiquement acceptable.

3. Compositions selon la revendication 2, caractérisées en ce qu'elles contiennent à titre de principe actif un composé de formule I dans laquelle Z est un groupe thiazolyle-2, $R_1$ est un groupe méthyle et $R_2$ est un groupe thiényle,
ou l'un de ses sels d'addition avec un acide pharmaceutiquement acceptable.

4. Compositions selon la revendication 1, caractérisées en ce qu'elles contiennent à titre de principe actif un composé de formule I dans laquelle Z est un groupe phényle, $R_1$ représente un groupe de formule

$-NHR_3$ ou $-NR_4R_5$ et $R_2$ représente un groupe aryle ou hétéroaromatique,
ou l'un de ses sels d'addition avec un acide pharmaceutiquement acceptable.

5. Compositions selon la revendication 4, caractérisées en ce qu'elles contiennent à titre de principe actif un composé de formule I dans laquelle Z représente un groupe phényle, $R_1$ est un groupe amino ou méthylpipérazinyle et $R_2$ est un groupe phényle
ou l'un de ses sels d'addition avec un acide pharmaceutiquement acceptable.

6. Compositions selon l'une quelconque des revendications 1 à 5, caractérisées en ce qu'elles sont sous forme d'un collyre.

7. Composés de formule

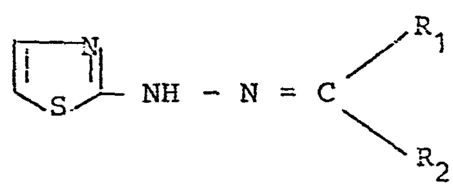

(I)

dans laquelle

$R_1$ représente un atome d'hydrogène, un groupe alkyle en $C_1$-$C_6$, un groupe cycloalkyle en $C_5$-$C_7$, un groupe hydroxy, un groupe alkoxy en $C_1$-$C_6$, un atome d'halogène, un groupe amino ou un groupe (alkyl en $C_1$-$C_6$) amino,

$R_2$ représente un groupe carboxy (alkyle en $C_1$ à $C_6$), un groupe (alkoxy en $C_1$-$C_6$) carbonyl (alkyle en $C_1$-$C_6$), un groupe thiényle, un groupe furyle, un groupe imidazolyl-1 (alkyle en $C_1$-$C_{10}$), un groupe phényle substitué par un ou plusieurs groupes hydroxy,
ou bien

$R_1$ et $R_2$ forment avec l'atome de carbone sur

lequel ils sont fixés un radical choisi parmi les radicaux hydrocarbonés aromatiques bi- ou polycycliques ayant de 8 à 16 atomes de carbone, les radicaux hydrocarbonés mono- ou polycycliques non aromatiques ayant de 5 à 16 atomes de carbone et les radicaux hétéroaromatiques bi- ou polycycliques comprenant un ou deux hétéroatomes choisis parmi l'azote, l'oxygène et le soufre et de 6 à 15 atomes de carbone,
ainsi que leurs sels d'addition avec des acides pharmaceutiquement acceptables.

FIG.1

MÉTABOLISME DE L'ACIDE ARACHIDONIQUE

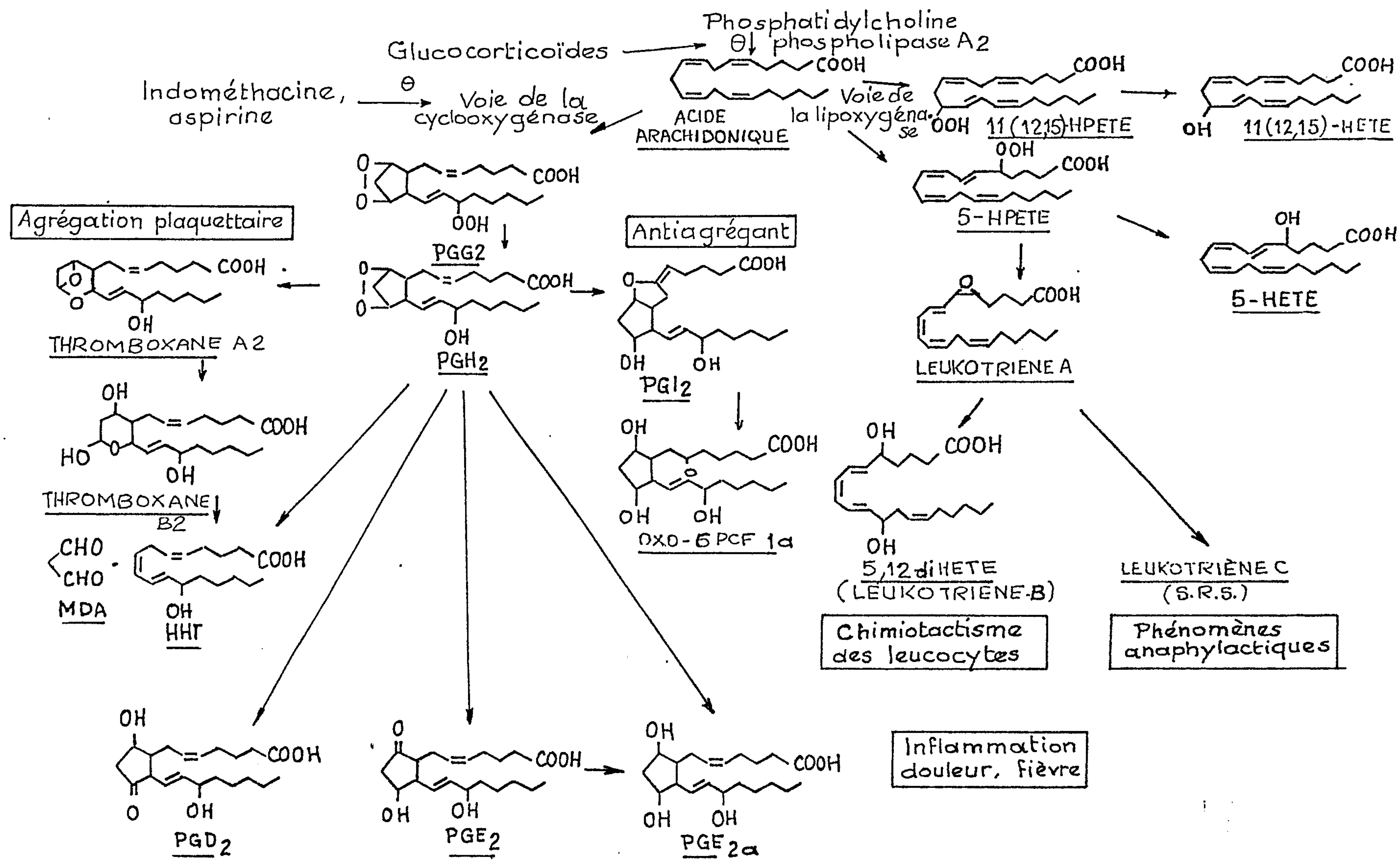

FIG. 2b

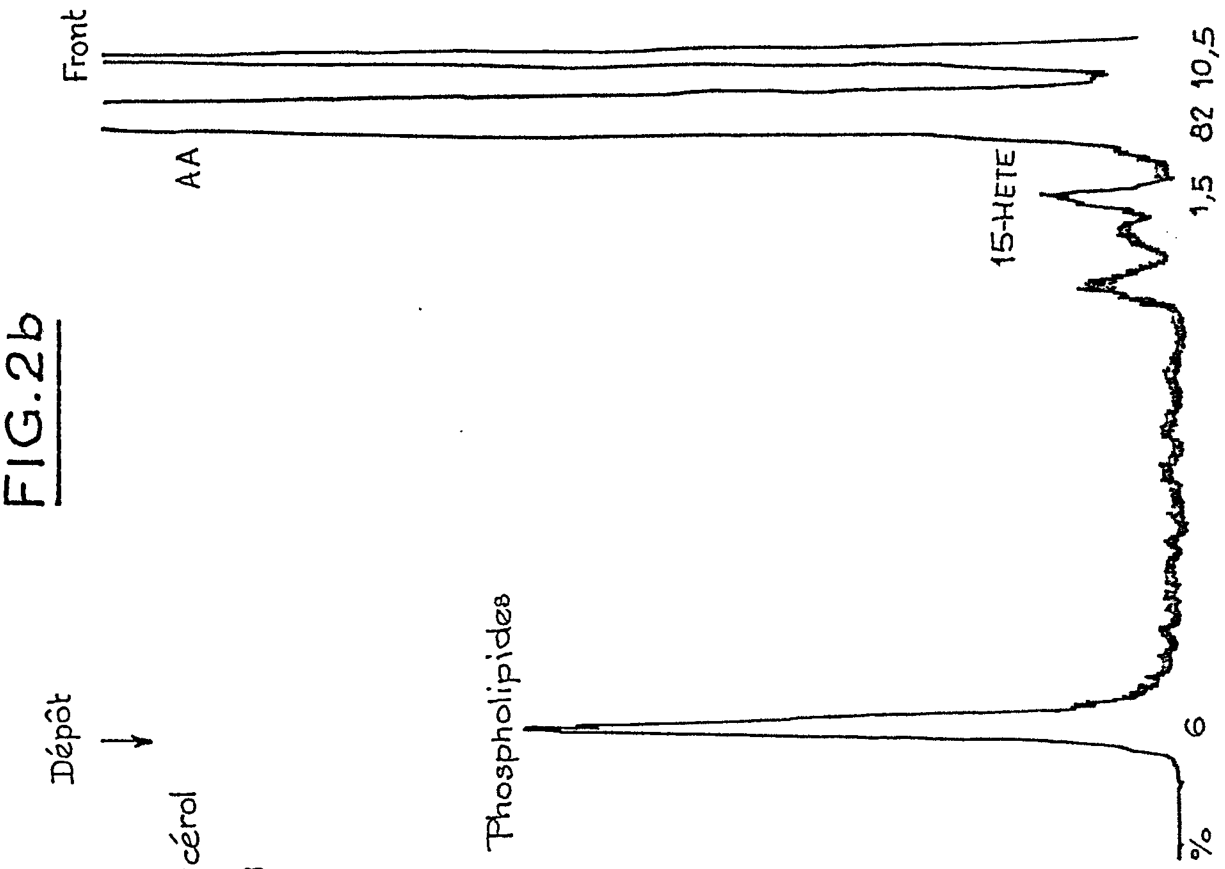
Front
AA
15-HETE
10,5
82
1,5
Dépôt
Phospholipides
6
%

FIG. 2a

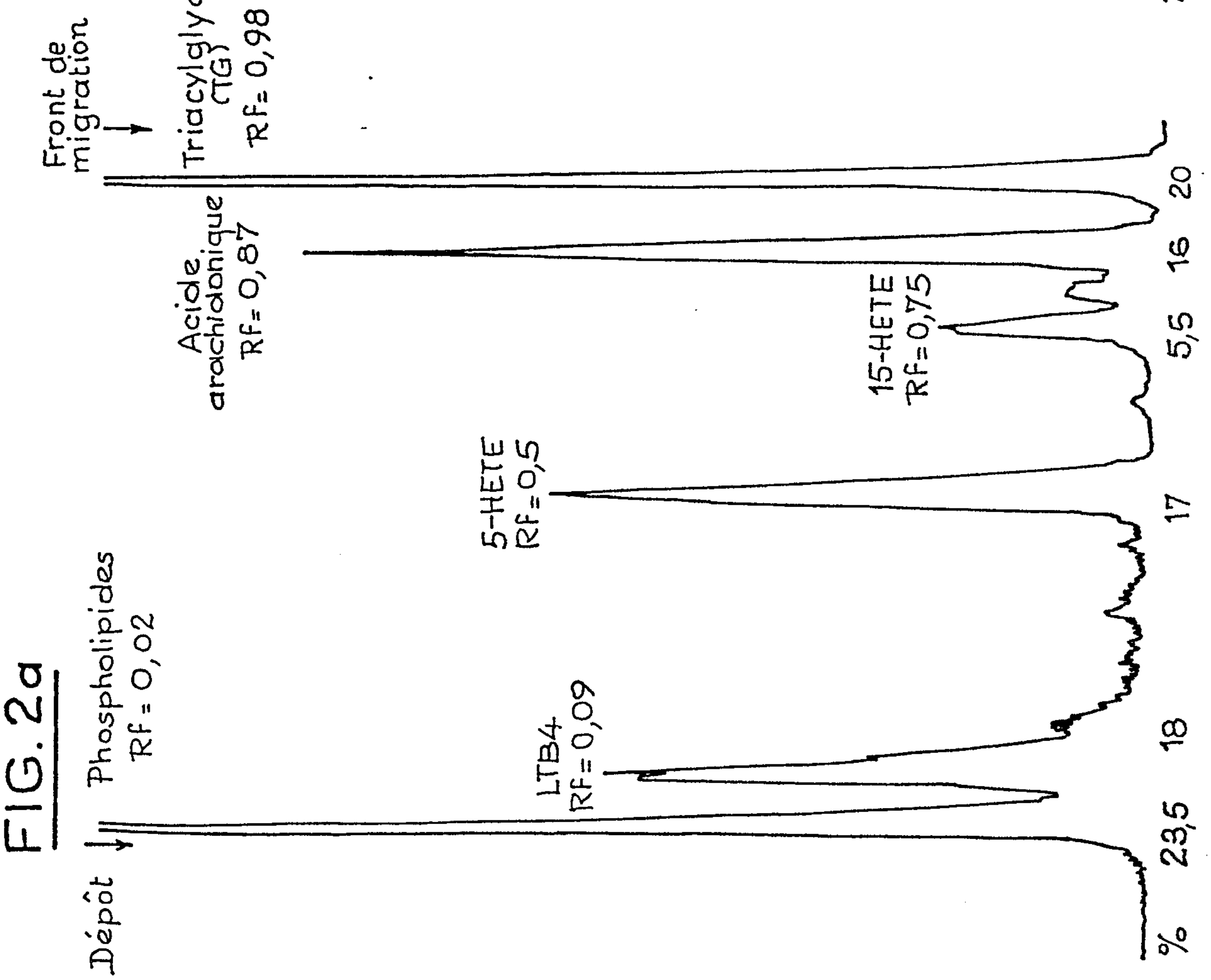
Front de migration
Triacylglycérol (TG) Rf= 0,98
Acide arachidonique Rf= 0,87
15-HETE Rf= 0,75
5-HETE Rf= 0,5
LTB4 Rf= 0,09
Phospholipides Rf = 0,02
Dépôt
20
16
5,5
17
18
23,5
%

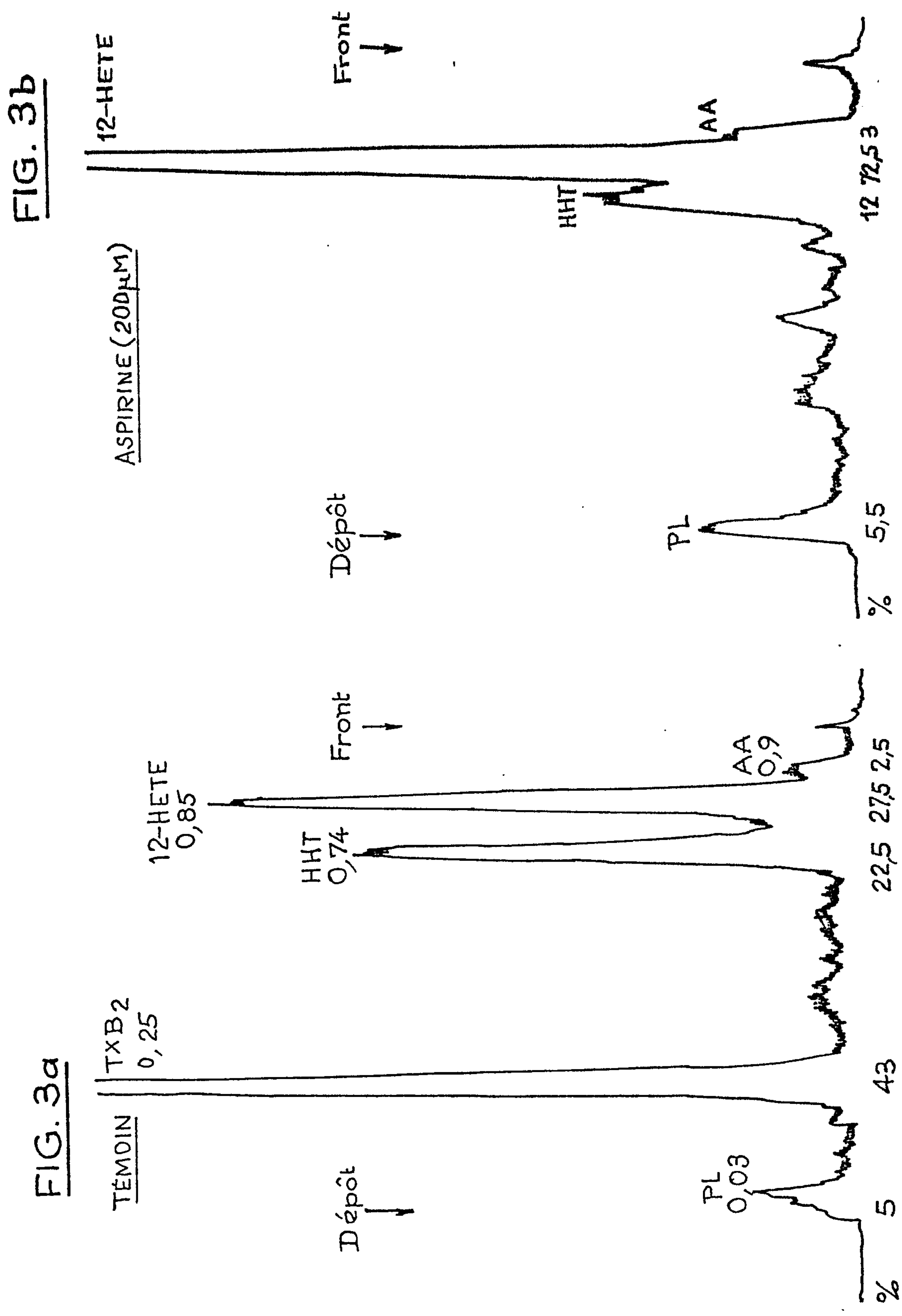
FIG. 3b
ASPIRINE (200µM)
12-HETE
Front
AA
HHT
12 72,5 3
Dépôt
PL
5,5
%
FIG. 3a
TEMOIN
TXB2
0,25
12-HETE
0,85
HHT
0,74
Front
AA
0,9
22,5 27,5 2,5
43
Dépôt
PL
0,03
5
%

FIG. 4b

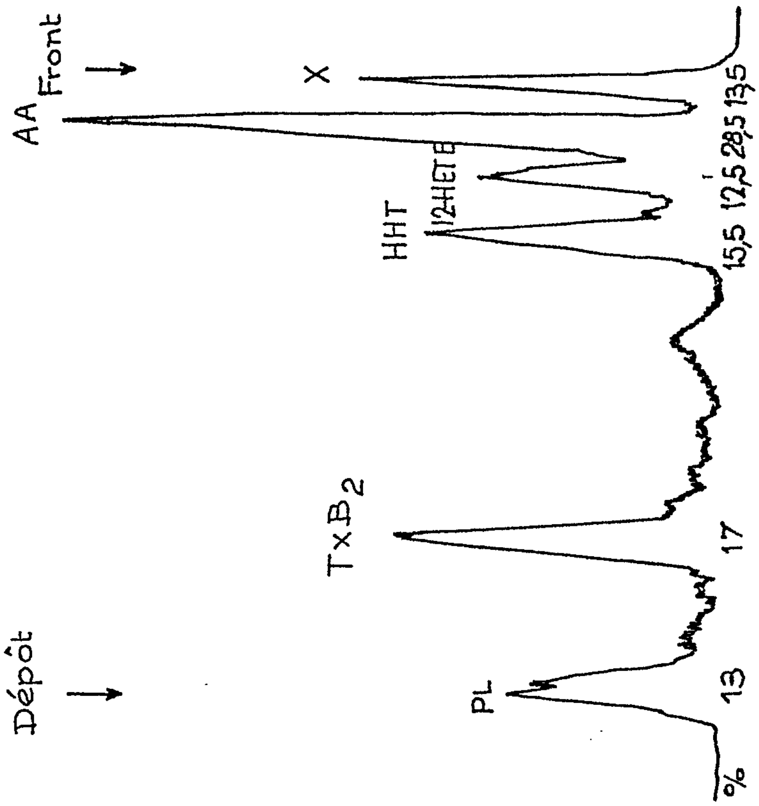

%
Dépôt
PL
13
TxB $_2$
17
15,5
HHT
12HETE
12,5
28,5
13,5
AA
Front
X


FIG. 4a

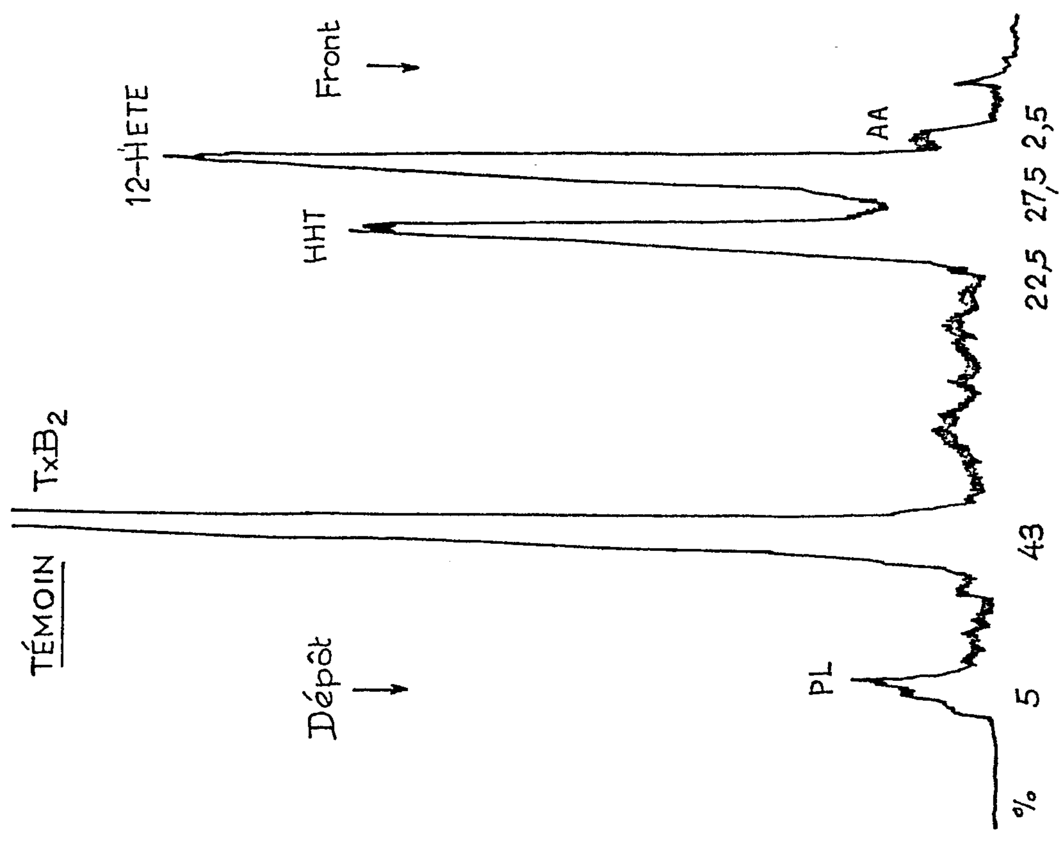

TÉMOIN
%
Dépôt
PL
5
43
TxB $_2$
22,5
HHT
27,5
12-HETE
2,5
AA
Front

0098204

Office européen des brevets

## RAPPORT DE RECHERCHE EUROPEENNE

Numéro de la demande

EP 83 40 1266

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,X | CHEMISCHE BERICHTE, vol. 85, no. 12, 1952, pages 1122-1129, Weinheim, DE. H. BEYER et al.: "Über Thiazole, XII. Mitteilung: Synthesen von Thiazolyl-(2)-hydrazinen" * En entier * --- | 7 | A 61 K 31/425<br>A 61 K 31/15<br>A 61 K 31/155<br>A 61 K 31/435<br>A 61 K 31/495<br>A 61 K 31/535<br>C 07 D 277/50<br>C 07 D 417/12<br>C 07 D 417/14 |
| D,X | CHEMISCHE BERICHTE, vol. 89, no. 5, mai 1956, pages 1095-1099, Weinheim, DE. H. BEYER et al.: "Über Thiazole, XXVII. Mitteil.: Zur Kenntnis der Thiazolyl-(2)-hydrazone" * En entier * --- | 7 | |
| D,X | TALANTA, vol. 27, no. 1, 1980, pages 55-58, Pergamon Press Ltd., Oxford, GB. A.A. SCHILT et al.: "New chromogens of the ferroin type-X. Synthesis and chelation properties of some 2-thiazolyl- and 2-pyrimidinylhydrazones" * En entier * --- -/- | 7 | DOMAINES TECHNIQUES RECHERCHES (Int. Cl. 3)<br>C 07 D 277/00<br>C 07 D 417/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 25-08-1983 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503.03.82

Office européen des brevets

# RAPPORT DE RECHERCHE EUROPEENNE

Numéro de la demande

EP 83 40 1266



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,A | BULLETIN DE LA SOCIETE CHIMIQUE DE FRANCE, no. 9-10, septembre-octobre 1973, pages 2843-2847, Paris, FR. J. PERRONNET et al.: "No. 528. - Réactivité des chlorures d'acides N-aryl-benzhydrazoniques" * En entier * --- | 1 | |
| A | FR-A-2 105 698 (ROUSSEL-UCLAF) * Revendications * --- | 1 | |
| A | CHEMICAL ABSTRACTS, vol. 73, no. 16, 19 octobre 1970, page 66, no. 78537g, Columbus, Ohio, USA & JP - A - 70 12594 (NIPPON KAYAKU CO., LTD.) 07-05-1970 * Résumé * ----- | 7 | |
| | | | DOMAINES TECHNIQUES RECHERCHES (Int. Cl. 3) |

Le présent rapport de recherche a été établi pour toutes les revendications

| Lieu de la recherche | Date d'achèvement de la recherche | Examinateur |
|---|---|---|
| LA HAYE | 25-08-1983 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503. 03.82